# EUROPEAN PATENT APPLICATION

(11) **EP 4 371 988 A1**
(43) Date of publication of application: **22.05.2024**
(21) Application number: 22841960.2
(22) Date of filing: 30.06.2022
(51) Int. Cl.: C07D 493/10, C08K 5/1575, C08L 101/02, C09K 15/08

(54) **PHENOLIC COMPOUND, ORGANIC MATERIAL STABILIZER, RESIN COMPOSITION, AND METHOD FOR STABILIZING ORGANIC MATERIAL**

(30) Priority: 15.07.2021 JP 2021117275
(71) Applicant: Sumitomo Chemical Company, Limited, Tokyo 103-6020 (JP)
(72) Inventor: KITAMURA, Kazuhiro, Osaka-shi, Osaka 554-8558 (JP)
(74) Representative: Vossius & Partner Patentanwälte Rechtsanwälte mbB
(86) International application number: PCT/JP2022/026154
(87) International publication number: WO 2023/286616

(57) **Abstract**

The present invention relates to a phenolic compound represented by the following Formula (1) [wherein, R¹ represents an alkyl group having 1 to 2 carbon atoms] :

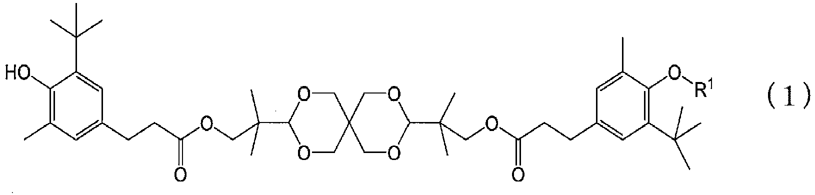

## Description

### TECHNICAL FIELD

The present invention relates to a novel phenolic compound, and the use thereof as an organic material stabilizer.

### BACKGROUND ART

It is known that organic materials, such as thermoplastic resins, thermosetting resins, natural or synthetic rubbers, mineral oils, lubricants, adhesives, and paints, are deteriorated by the action of heat, oxygen, or the like during production, processing, and use, and that the commercial values of such organic materials may be greatly impaired in association with deterioration in strength and physical properties, change in flowability, coloring, deterioration of surface properties, and the like that are caused by phenomena such as molecular cleavage and molecular crosslinking.

For the purpose of preventing such deterioration by heat or oxygen, various phenolic antioxidants and phosphorus-based antioxidants have been previously developed, and it is known that organic materials can be stabilized by adding these antioxidants to the organic materials (Patent Literature 1). However, those antioxidants that are conventionally used may be insufficient in terms of the stabilization effect against deterioration caused by heat or oxygen during processing of an organic material, and a compound having a superior stabilization effect is desired.

### PRIOR ART DOCUMENTS

### PATENT DOCUMENTS

Patent Document 1: JP H08-002998 A

### SUMMARY OF THE INVENTION

### PROBLEMS TO BE SOLVED BY THE INVENTION

An object of the present invention is to provide a novel compound that is effective in improving the thermal stability during processing of an organic material.

### SOLUTIONS TO THE PROBLEMS

The present inventors investigated various compounds in detail so as to solve the above-described problem, and consequently discovered a novel phenolic compound, thereby completing the present invention.

That is, the present invention encompasses the following preferred aspects.
[1] A phenolic compound, represented by the following Formula (1): 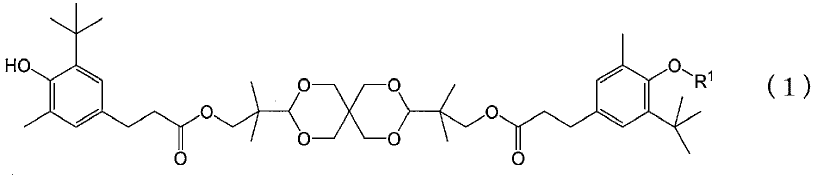 wherein, R¹ represents an alkyl group having 1 to 2 carbon atoms.
[2] The phenolic compound according to [1], wherein R¹ is a methyl group.
[3] An organic material stabilizer, comprising the phenolic compound according to [1] or [2].
[4] A resin composition, comprising:
   the phenolic compound according to [1] or [2], or the organic material stabilizer according to [3]; and
   at least one organic material.
[5] The resin composition according to [4], wherein the organic material is a thermoplastic resin.
[6] The resin composition according to [5], wherein the thermoplastic resin is a polyolefin or an engineering plastic.
[7] A method for stabilizing an organic material, comprising:
   adding the phenolic compound according to [1] or [2], or the organic material stabilizer according to [3], to the organic material.
[8] The method according to [7], wherein the organic material is a thermoplastic resin.
[9] The method according to [8], wherein the thermoplastic resin is a polyolefin or an engineering plastic.

### EFFECTS OF THE INVENTION

The phenolic compound of the present invention is effective in improving the thermal stability during processing of an organic material such as a thermoplastic resin.

### DETAILED DESCRIPTION

Embodiments of the present invention will now be described in detail. It is noted here, however, that the scope of the present invention is not limited to the embodiments described below, and various modifications can be made without departing from the gist of the present invention. Further, when plural upper limit values and lower limit values are stated for a specific characteristic, a combination of any of these upper limit values and lower limit values can be taken as a preferred numerical range.

The present invention provides a phenolic compound represented by the following Formula (1): 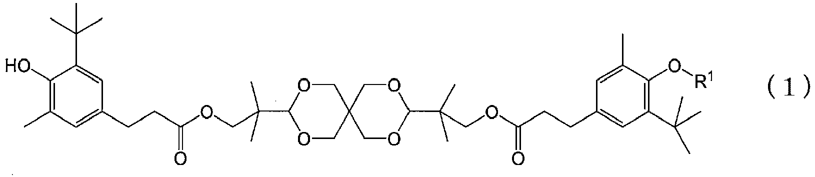 [wherein, R¹ represents an alkyl group having 1 to 2 carbon atoms].

In Formula (1), R¹ represents an alkyl group having 1 to 2 carbon atoms, specifically a methyl group or an ethyl group. From the standpoint of facilitating the stabilization of an organic material such as a thermoplastic resin, R¹ is preferably a methyl group.

The phenolic compound represented by Formula (1) can be produced by allowing a compound represented by Formula (1), in which the -OR¹ group moiety is an -OH group, and an alkyl halide containing an alkyl group having 1 to 2 carbon atoms (e.g., methyl iodide or ethyl iodide) to react with each other in the presence of nitrogen.

This reaction is usually performed in an organic solvent. The organic solvent is not particularly limited as long as it does not inhibit the reaction, and examples of the organic solvent include ketone-based solvents, ether-based solvents, aromatic hydrocarbon solvents, aliphatic hydrocarbon solvents, and halogenated hydrocarbon solvents. The reaction may be performed in a single kind of organic solvent, in a mixed solvent of two or more kinds of organic solvents, or in a mixed solvent of the above-described organic solvent(s) and other solvent.

Examples of the ketone-based solvents include acetone, methyl ethyl ketone, methyl isobutyl ketone, and cyclohexanone. Examples of the ether-based solvents include diethyl ether and dibutyl ether. Examples of the aromatic hydrocarbon solvents include benzene, toluene, xylene, and ethylbenzene. Examples of the aliphatic hydrocarbon solvents include n-hexane, n-heptane, and n-octane. Examples of the halogenated hydrocarbon solvents include chloroform, carbon tetrachloride, monochlorobenzene, dichloromethane, 1,2-dichloroethane, and dichlorobenzene.

The reaction is performed at room temperature.

In this method, it is preferred to use the alkyl halide containing an alkyl group having 1 to 2 carbon atoms in an amount of approximately 3 times by mole with respect to the compound represented by Formula (1) in which the -OR¹ group moiety is an -OH group. Further, potassium carbonate is preferably added in an amount of approximately 3 times by mole with respect to the compound represented by Formula (1) in which the -OR¹ group moiety is an -OH group.

A method of synthesizing the compound represented by Formula (1) in which the -OR¹ group moiety is an -OH group that is used as a raw material is known, and the compound can be synthesized in accordance with the method disclosed in, for example, Japanese Patent No. 3661198.

By adding the phenolic compound of the present invention that is represented by Formula (1) to an organic material, for example, thermal deterioration and oxidative deterioration of the organic material can be reduced and the organic material can thus be stabilized, so that a reduction in viscosity, coloring, and the like can be inhibited. Therefore, the phenolic compound of the present invention is suitable as an active ingredient of an organic material stabilizer. Accordingly, the present invention also provides: an organic material stabilizer containing the phenolic compound of the present invention that is represented by Formula (1); and a method for stabilizing an organic material, which method includes adding the phenolic compound of the present invention or the above-described organic material stabilizer to the organic material. The present invention further provides a stabilized resin composition that contains an organic material and the phenolic compound of the present invention or the organic material stabilizer of the present invention.

In the above-described aspect, as the phenolic compound of the present invention that is represented by Formula (1), a single kind of phenolic compound represented by Formula (1) may be used, or two or more kinds of phenolic compounds represented by Formula (1) may be used in combination.

Examples of the organic material that can be stabilized by the phenolic compound of the present invention include the following, but it is not limited to these organic materials. The organic material may be one kind of an organic material or a mixture of two or more kinds of organic materials.
(1) Polyethylene such as high density polyethylene (HD-PE), low density polyethylene (LD-PE), linear low density polyethylene (LLDPE),
(2) Polypropylene,
(3) Methylpentene polymer,
(4) EAA (ethylene / ethyl acrylate copolymer) resin,
(5) Ethylene / vinyl acetate copolymer resin,
(6) Polystyrenes such as polystyrene, poly(p-methylstyrene), poly (a-methylstyrene),
(7) AS (acrylonitrile/styrene copolymer) resin,
(8) ABS (acrylonitrile/butadiene/styrene copolymer) resin,
(9) AAS (special acrylic rubber/acrylonitrile/styrene copolymer) resin,
(10) ACS (acrylonitrile/chlorinated polyethylene/styrene copolymer) resin,
(11) Chlorinated polyethylene, polychloroprene, chlorinated rubber,
(12) Polyvinyl chloride, polyvinylidene chloride,
(13) Methacrylic resin,
(14) Ethylene/vinyl alcohol copolymer resin,
(15) Fluororesin,
(16) Polyacetal,
(17) Grafted polyphenylene ether resin and polyphenylene sulfide resin,
(18) Polyurethane,
(19) Polyamide,
(20) Polyester resin such as polyethylene terephthalate, polybutylene terephthalate,
(21) Polycarbonate,
(22) Polyacrylate,
(23) Polysulfone, polyether ether ketone, polyether sulphone,
(24) Thermoplastic resins, such as aromatic polyester resin,
(25) Epoxy resin,
(26) Diallyl phthalate prepolymer,
(27) Silicone resin,
(28) Unsaturated polyester resin,
(29) Acrylic modified benzoguanamine resin,
(30) Benzoguanamine/melamine resin,
(31) Thermosetting resins, such as urea resin,
(32) Polybutadiene,
(33) 1,2-Polybutadiene,
(34) Polyisoprene,
(35) Styrene/butadiene copolymer,
(36) Butadiene/acrylonitrile copolymer,
(37) Ethylene/propylene copolymer,
(38) Silicone rubber,
(39) Epichlorhydrin rubber,
(40) Acrylic rubber,
(41) Natural rubber,
(42) Chlorine rubber type paint,
(43) Polyester resin paint,
(44) Urethane resin paint,
(45) Epoxy resin paint,
(46) Acrylic resin paint,
(47) Vinyl resin paint,
(48) Amino alkyd resin paint,
(49) Alkyd resin paint,
(50) Nitrocellulose resin paint,
(51) Oil paint,
(52) Wax,
(53) Lubricating oil and so on.

Among them, thermoplastic resins, particularly polyethylene, for example, polyolefins such as HD-PE, LD-PE, LLDPE and polypropylene, engineering plastics such as polyamide, polyethylene terephthalate, polybutylene terephthalate and polycarbonate, and the like are preferably used.

The polyolefin is not particularly limited, and may be, for example, one obtained by radical polymerization or may be one produced by polymerization using a catalyst containing a metal of group IVb, Vb, VIb or VIII of the periodic table. Examples of the catalyst containing such a metal include a metal complex having one or more ligands, for example, an oxide, a halogen compound, an alcoholate, an ester, and an aryl coordinated by a π or σ bond. These complexes may be used as they are, or they may be supported on a substrate such as magnesium chloride, titanium chloride, alumina, silicon oxide or the like. As the polyolefin, for example, one produced by using a Ziegler-Natta catalyst, a TNZ catalyst, a metallocene catalyst, a Phillips catalyst or the like is preferably used.

Engineering plastics are also not particularly limited. The polyamide resin is one having an amide bond in the polymer chain, and may be anything as long as it can be heated and melted. The polyamide resin may be produced by any method, for example, one produced by a condensation reaction of a diamine and a dicarboxylic acid, a condensation reaction of an aminocarboxylic acid, a ring-opening polymerization of a lactam or the like. Examples of the polyamide resin include nylon 66, nylon 69, nylon 610, nylon 612, poly-bis-(p-aminocyclohexyl)methandodecamide, nylon 46, nylon 6, nylon 12, Nylon 66/6 which is a copolymer of nylon 66 and nylon 6, nylon 6/12 and the like. The polyester resin is one having an ester bond in the polymer chain and may be anything as long as it can be heated and melted. Examples thereof include a polyester obtained by polycondensation of a dicarboxylic acid and a dihydroxy compound or the like. The polyester resin may be either a homopolyester or a copolyester. The polycarbonate resin is one having a carbonate bond in the polymer chain and may be anything as long as it can be heated and melted. Examples thereof include a polycarbonate resin obtained by reacting an aromatic hydroxy compound or additionally to this a small amount of a polyhydroxy compound with a carbonate precursor such as phosgene or diphenyl carbonate in the presence of a solvent, an acid acceptor, or a molecular weight regulator. The polycarbonate resin may be linear, branched, or may be a copolymer.

When the organic material is stabilized by adding the phenolic compound of the present invention represented by Formula (1) to the organic material, the content of the phenolic compound of the present invention is, from the viewpoint of stabilizing the organic material, usually 0.001 part by mass or more, preferably 0.01 part by mass or more, more preferably 0.05 part by mass or more, based on 100 parts by mass of the organic material. The content of the phenolic compound of the present invention is usually 5 parts by mass or less, preferably 3 parts by mass or less, more preferably 1 part by mass or less, based on 100 parts by mass of the organic material from the viewpoint of efficiently stabilizing the organic material and from the economical viewpoint.

In adding the phenolic compound of the present invention represented by Formula (1) to the organic material, further additives can be added to the organic material as needed, and examples of the additive include phenolic antioxidants, sulfur-based antioxidants, phosphorus antioxidants, ultraviolet absorbers, light stabilizers, peroxide scavengers, polyamide stabilizers, hydroxylamines, lubricants, plasticizers, flame retardants, nucleating agents, metal deactivators, antistatic agents, pigments, fillers, pigments, antiblocking agents, surfactants, processing aids, foaming agents, emulsifiers, brighteners, calcium stearate, neutralizers such as hydrotalcite, further, color improvers such as 9,10-dihydro-9-oxa-10-phosphophenanthrene-10-oxide, supplementary stabilizers such as benzofurans and indolines described in U.S. Patent No. 4,325,853, U.S. Patent No. 4,338,244, U.S. Patent No. 5,175,312, U.S. Patent No. 5,216,053, U.S. Patent No. 5,252,643, U.S. Patent No. 4,316,611, DE-A-4,316,622, DE-A-4,316,876, EP-A-589,839, and EP-A-591,102, and the like. These additives can be added to the organic material simultaneously with the phenolic compound of the present invention or can be added to the organic material at a stage different from the phenolic compound of the present invention. As the additives, one kind of additives may be used, or two or more kinds of additives may be used in combination.

Examples of the phenolic antioxidant include the followings. As the phenolic antioxidant, the following compounds may be used alone, or two or more of them may be used in combination.

### (1) Examples of alkylated monophenols

2,6-Di-t-butyl-4-methylphenol, 2,4,6-tri-t-butylphenol, 2,6-di-t-butylphenol, 2-t-butyl-4,6-dimethylphenol, 2,6-di-t-butyl-4-ethylphenol, 2,6-di-t-butyl-4-n-butylphenol, 2,6-di-t-butyl-4-isobutylphenol, 2,6-dicyclopentyl-4-methylphenol, 2-(α-methylcyclohexyl)-4,6-dimethylphenol, 2,6-dioctadecyl-4-methylphenol, 2,4,6-tricyclohexylphenol, 2,6-di -t-butyl-4-methoxymethylphenol, 2,6-di-nonyl-4-methylphenol, 2,4-dimethyl-6-(1'-methylundecyl-1'-yl)phenol, 2,4-dimethyl-6-(1'-methylheptadecyl-1'-yl)phenol, 2,4-dimethyl-6-(1'-methyl tridecyl-1'-yl)phenol and any mixtures thereof.

### (2) Examples of alkylthiomethylphenols

2,4-Dioctylthiomethyl-6-t-butylphenol, 2,4-dioctylthiomethyl-6-methylphenol, 2,4-dioctylthiomethyl-6-ethylphenol, 2,6-didodecylthiomethyl-4-nonylphenol and any mixtures thereof.

### (3) Examples of hydroquinone and alkylated hydroquinones

2,6-Di-t-butyl-4-methoxyphenol, 2,5-di-t-butylhydroquinone, 2,5-di-t-amylhydroquinone, 2,6-diphenyl-4-octadecyloxyphenol, 2,6-di-t-butylhydroquinone, 2,5-di-t-butyl-4-hydroxyanisole, 3,5-di-t-butyl-4-hydroxyphenylstearate, bis(3,5-di-t-butyl-4-hydroxyphenyl)adipate and any mixtures thereof.

### (4) Examples of tocopherols

α-Tocopherol, β-tocopherol, γ-tocopherol, δ-tocopherol and any mixtures thereof.

### (5) Examples of hydroxylated thiodiphenyl ethers

2,2'-Thiobis(6-t-butylphenol), 2,2'-thiobis(4-methyl-6-t-butylphenol), 2,2'-thiobis(4-octylphenol), 4,4'-thiobis(3-methyl-6-t-butylphenol), 4,4'-thiobis(2-methyl-6-t-butylphenol), 4,4'-thiobis(3,6-di-t-amylphenol), 4,4'-(2,6-dimethyl-4-hydroxyphenyl)disulfide and the like.

### (6) Examples of alkylidene bisphenols and derivatives thereof

2,2'-Methylenebis(4-methyl-6-t-butylphenol), 2,2'-methylenebis(4-ethyl-6-t-butylphenol), 2,2'-methylenebis[4-methyl-6-(α-methylcyclohexyl) phenol]], 2,2'-methylenebis(4-methyl-6-cyclohexylphenol), 2,2'-methylenebis(4-methyl-6-nonylphenol), 2,2'-methylenebis(4,6-di-t-butylphenol), 2,2'-ethylidenebis(4,6-di-t-butylphenol), 2,2'-ethylidenebis(4-isobutyl-6-t-butylphenol), 2,2'-methylenebis[6-(α-methylbenzyl)-4-nonylphenol], 2,2'-methylenebis[4,6-(α,α-dimethylbenzyl)-4-nonylphenol], 4,4'-methylenebis(6-t-butyl-2-methylphenol), 4,4'-methylenebis(2,6-di-t-butylphenol), 4,4'-butylidenebis(3-methyl-6-t-butylphenol), 1,1-bis(4-hydroxyphenyl)cyclohexane, 1,1-bis(5-t-butyl-4-hydroxy-2-methylphenyl)butane, 2,6-bis(3-t-butyl-5-methyl-2-hydroxybenzyl)-4-methylphenol, 1,1,3-tris(5-t-butyl-4-hydroxy-2-methylphenyl) butane, 1,1-bis(5-t-butyl-4-hydroxy-2-methylphenyl)-3-n-dodecylmercaptobutane, ethyleneglycolbis[3,3-bis-3'-butyl-4'-hydroxyphenyl)butyrate], bis(3-t-butyl-4-hydroxy-5-methylphenyl)dicyclopentadiene, bis[2-(3'-t-butyl-2'-hydroxy-5'-methylbenzyl)-6-t-butyl-4-methylphenyl]terephthalate, 1,1-bis(3,5-dimethyl-2-hydroxyphenyl)butane, 2,2-bis(3,5-di-t-butyl-4-hydroxyphenyl)propane, 2,2-bis(5-t-butyl-4-hydroxy-2-methylphenyl)-4-n-dodecylmercaptobutane, 1,1,5,5-tetra(5-t-butyl-4-hydroxy-2-methylphenyl)pentane, 2-t-butyl-6-(3'-t-butyl-5'-methyl-2'-hydroxybenzyl)-4-methylphenylacrylate, 2,4-di-t-pentyl-6-[1-(2-hydroxy-3,5-di-t-pentylphenyl)ethyl]phenylacrylate and any mixtures thereof.

### (7) Examples of O-, N- and S-benzyl derivatives

3,5,3',5'-Tetra-t-butyl-4,4'-dihydroxydibenzylether, octadecyl-4-hydroxy-3,5-dimethylbenzylmercaptoacetate, tris(3,5-di-t-butyl-4-hydroxybenzyl)amine, bis(4-t-butyl-3-hydroxy-2,6-dimethylbenzyl) dithio terephthalate, bis(3,5-di-t-butyl-4-hydroxybenzyl)sulfide, isooctyl-3,5-di-t-butyl-4-hydroxybenzylmercaptoacetate and any mixtures thereof.

### (8) Examples of hydroxybenzylated malonate derivatives

Dioctadecyl-2,2-bis(3,5-di-t-butyl-2-hydroxybenzyl)malonate, dioctadecyl 2-(3-t-butyl-4-hydroxy-5-methylbenzyl)malonate, didodecylmercaptoethyl-2,2-bis(3,5-dit-butyl-4-hydroxybenzyl)malonate, bis[4-(1,1,3,3 -tetramethylbutyl)phenyl] -2,2-bis(3,5-di-t-butyl-4-hydroxybenzyl) malonate and any mixtures thereof.

### (9) Examples of aromatic hydroxybenzyl derivatives

1,3,5-Trimethyl-2,4,6-tris(3,5-di-t-butyl-4-hydroxybenzyl)benzene, 1,4-bis(3,5-di-t-butyl-4-hydroxybenzyl)-2,3,5,6-tetramethylbenzene, 2,4,6-tris(3,5-t-butyl-4-hydroxybenzyl)phenol and any mixtures thereof.

### (10) Examples of triazine derivatives

2,4-Bis(n-octylthio)-6-(4-hydroxy-3,5-di-t-butylanilino)-1,3,5-triazine, 2-n-octylthio-4,6-bis(4-hydroxy-3,5-di-t-butylanilino)-1,3,5-triazine, 2-n-octylthio-4,6-bis(4-hydroxy-3,5-di-t-butylphenoxy)1,3,5-triazine, 2,4,6-tris(3,5-di-t-butyl-4-phenoxy)-1,3,5-triazine, tris(4-t-butyl-3-hydroxy-2,6-dimethylbenzyl)isocyanurate, tris(3,5-di-t-butyl-4-hydroxybenzyl)isocyanurate, 2,4,6-tris(3,5-di-t-butyl-4-hydroxyphenylethyl)-1,3,5-triazine, 2,4,6-tris(3,5-di-t-butyl-4-hydroxyphenylpropyl)-1,3,5-triazine, tris(3,5-dicyclohexyl-4-hydroxybenzyl)isocyanurate, tris[2-(3',5'-di-t-butyl-4'-hydroxycinnamoyloxy)ethyl] isocyanurate and any mixtures thereof.

### (11) Examples of benzylphosphonate derivatives

Dimethyl-3,5-di-t-butyl-4-hydroxybenzylphosphonate, diethyl-3,5-di-t-butyl-4-hydroxybenzylphosphonate, dioctadecyl-3,5-di-t-butyl-4-hydroxybenzylphosphonate, dioctadecyl-5-t-butyl-4-hydroxy-3-methylbenzylphosphonate, calcium salt of 3,5-di-t-butyl-4-hydroxybenzylphosphonic acid monoester and any mixtures thereof.

### (12) Examples of acylaminophenol derivatives

4-Hydroxyl lauric acid anilide, 4-hydroxystearic acid anilide, octyl-N-(3,5-di-t-butyl-4-hydroxyphenyl)carbanate and any mixtures thereof.

### (13) Examples of esters of β-(3,5-di-t-butyl-4-hydroxyphenyl)propionic acid with the following monohydric or polyhydric alcohols

Methanol, ethanol, octanol, octadecanol, ethylene glycol, 1,3-propanediol, 1,4-butanediol, 1,6-hexanediol, 1,9-nonanediol, neopentyl glycol, diethylene glycol, thioethylene glycol , spiroglycol, triethylene glycol, pentaerythritol, tris(hydroxyethyl)isocyanurate, N,N'-bis(hydroxyethyl)oxamide, 3-thiaundecanol, 3-thiapentadecanol, trimethylhexanediol, trimethylol propane, 4-hydroxymethyl-1-phospha-2,6,7-trioxabicyclo[2,2,2]octane and any mixtures thereof.

### (14) Examples of esters of β-(5-t-butyl-4-hydroxy-3-methylphenyl)propionic acid with the following monohydric or polyhydric alcohols

Methanol, ethanol, octanol, octadecanol, ethylene glycol, 1,3-propanediol, 1,4-butanediol, 1,6-hexanediol, 1,9-nonanediol, neopentyl glycol, diethylene glycol, thioethylene glycol , spiroglycol, triethylene glycol, pentaerythritol, tris(hydroxyethyl)isocyanurate, N,N'-bis(hydroxyethyl)oxamide, 3-thiaundecanol, 3-thiapentadecanol, trimethylhexanediol, trimethylol propane, 4-hydroxymethyl-1-phospha-2,6,7-trioxabicyclo[2,2,2]octane and any mixtures thereof.

### (15) Examples of esters of β-(3,5-dicyclohexyl-4-hydroxyphenyl)propionic acid with the following monohydric or polyhydric alcohols

Methanol, ethanol, octanol, octadecanol, ethylene glycol, 1,3-propanediol, 1,4-butanediol, 1,6-hexanediol, 1,9-nonanediol, neopentyl glycol, diethylene glycol, thioethylene glycol , spiroglycol, triethylene glycol, pentaerythritol, tris(hydroxyethyl)isocyanurate, N,N'-bis(hydroxyethyl)oxamide, 3-thiaundecanol, 3-thiapentadecanol, trimethylhexanediol, trimethylol propane, 4-hydroxymethyl-1-phospha-2,6,7-trioxabicyclo[2,2,2]octane and any mixtures thereof.

### (16) Examples of esters of 3,5-di-t-butyl-4-hydroxyphenyl acetic acid with the following monohydric or polyhydric alcohols

Methanol, ethanol, octanol, octadecanol, ethylene glycol, 1,3-propanediol, 1,4-butanediol, 1,6-hexanediol, 1,9-nonanediol, neopentyl glycol, diethylene glycol, thioethylene glycol , spiroglycol, triethylene glycol, pentaerythritol, tris(hydroxyethyl)isocyanurate, N,N'-bis(hydroxyethyl)oxamide, 3-thiaundecanol, 3-thiapentadecanol, trimethylhexanediol, trimethylol propane, 4-hydroxymethyl-1-phospha-2,6,7-trioxabicyclo[2,2,2]octane and any mixtures thereof.

### (17) Examples of amides of β-(3,5-di-t-butyl-4-hydroxyphenyl)propionic acid

N,N'-bis[3-(3',5'-di-t-butyl-4'-hydroxyphenyl)propionyl]hydrazine, N,N'-bis[3-(3',5'-di-t-butyl-4'-hydroxyphenyl)propionyl]hexamethylenediamine, N,N'-bis[3-(3',5'-di-t-butyl-4'-hydroxyphenyl)propionyl]trimethylenediamine and any mixtures thereof.

Examples of the sulfur-based antioxidant include the followings.

Dilauryl 3,3'-thiodipropionate, tridecyl 3,3'-thiodipropionate, dimyristyl 3,3'-thiodipropionate, distearyl 3,3'-thiodipropionate, laurylstearyl 3,3'-thiodipropionate, neopentanetetrayltetrakis(3-laurylthiopropionate) and the like.

Examples of the phosphorus antioxidant include the followings. As the phosphorus antioxidant, the following compounds may be used alone, or two or more of them may be used in combination.

Triphenyl phosphite, tris(nonylphenyl)phosphite, tris(2,4-di-t-butylphenyl)phosphite, trilaurylphosphite, trioctadecylphosphite, distearyl pentaerythritol diphosphite, diisodecyl pentaerythritol diphosphite, bis(2,4-di-t-butylphenyl)pentaerythritol diphosphite, bis(2,4-di-t-butyl-6-methylphenyl)pentaerythritol diphosphite, bis(2,6-di-t-butyl-4-methylphenyl)pentaerythritol diphosphite, bis(2,4,6-tri-t-butylphenyl)pentaerythritol diphosphite, tristearylsorbitol triphosphite, tetrakis(2,4-di-t-butylphenyl)-4,4'-diphenylenediphosphonite, 2,2'-methylenebis(4,6-di-t-butylphenyl) 2-ethylhexyl phosphite, 2,2'-ethylidenebis(4,6-di-t-butylphenyl)fluorophosphite, bis(2,4-di-t-butyl-6-methylphenyl)ethyl phosphite, bis(2,4-di-t-butyl-6-methylphenyl)methyl phosphite, 2-(2,4,6-tri-t-butylphenyl)-5-ethyl-5-butyl-1,3,2-oxaphosphorinane, 2,2',2"-nitrilo[triethyl-tris(3,3',5,5'-tetra-t-butyl-1,1'-biphenyl-2,2'-diyl)phosphite, 6-[3-(3-t-butyl-4-hydroxy-5-methylphenyl)propoxy]-2,4,8,10-tetra-t-butyldibenz[d,f][1,3,2]dioxaphosphepin and any mixtures thereof.

Examples of the ultraviolet absorber include the followings. As the ultraviolet absorber, the following compounds may be used alone, or two or more of them may be used in combination.

### (1) Examples of salicylate derivatives

Phenyl salicylate, 4-t-butylphenyl salicylate, 2,4-di-t-butylphenyl 3',5'-di-t-butyl-4'-hydroxybenzoate, 4-t-octylphenyl salicylate, bis(4-t-butylbenzoyl)resorcinol, benzoyl resorcinol, hesadecyl 3',5'-di-t-butyl-4'-hydroxybenzoate, octadecyl 3',5'-di-t-butyl-4'-hydroxybenzoate, 2-methyl-4,6-di-t-butylphenyl 3',5'-di-t-butyl-4'-hydroxybenzoate and any mixtures thereof.

### (2) Examples of 2-hydroxybenzophenone derivatives

2,4-Dihydroxybenzophenone, 2-hydroxy-4-methoxybenzophenone, 2-hydroxy-4-octoxybenzophenone, 2,2'-dihydroxy-4-methoxybenzophenone, bis(5-benzoyl-4-hydroxy-2-methoxyphenyl)methane, 2,2',4,4'-tetrahydroxybenzophenone and any mixtures thereof.

### (3) Examples of 2-(2'-hydroxyphenyl)benzotriazole

2-(2'-hydroxy-5-methylphenyl)benzotriazole, 2-(3',5'-di-t-butyl-2'-hydroxyphenyl)benzotriazole, 2-(5'-t-butyl-2'-hydroxyphenyl)benzotriazole, 2-(2'-hydroxy-5'-t-octylphenyl)benzotriazole, 2-(3-t-butyl-2-hydroxy-5-methylphenyl)-5-chlorobenzotriazole,2-(3'-s-butyl-2'-hydroxy-5'-t-butylphenyl)benzotriazole, 2-(2'-hydroxy-4'-octyloxyphenyl)benzotriazole, 2-(3',5'-di-t-amyl-2'-hydroxyphenyl)benzotriazole, 2-[2'-hydroxy-3',3'-bis(α,α-dimethylbenzyl)phenyl]-2H-benzotriazole, 2-[(3'-t-butyl-2'-hydroxyphenyl)-5'-(2-octyloxycarbonylethyl)phenyl]-5-chlorobenzotriazole, 2-[3'-t-butyl-5'-[2-(2-ethylhexyloxy)carbonylethyl]-2'-hydroxyphenyl]-5-chlorobenzotriazole, 2- [3 '-t-butyl-3 '-hydroxy-5 '-(2-methoxycarbonylethyl)phenyl]-5-chlorobenzotriazole, 2-[3'-t-butyl-2'-hydroxy-5'-(2-methoxycarbonylethyl)phenyl]benzotriazole, 2-[3'-t-butyl-2'-hydroxy-5-(2-octyloxycarbonylethyl)phenyl]benzotriazole, 2-[3'-t-butyl-2'-hydroxy-5'-[2-(2-ethylhexyloxy)carbonylethyl]phenyl] benzotriazole, 2-[2-hydroxy-3-(3,4,5,6-tetrahydrophthalimidomethyl)-5-methylphenyl]benzotriazole, 2-(3,5-di-t-butyl-2-hydroxyphenyl)-5-chlorobenzotriazole, a mixture of 2-(3'-dodecyl-2'-hydroxy-5'-methylphenyl)benzotriazole and 2-[3'-t-butyl-2'-hydroxy-5'-(2-isooctyloxycarbonylethyl)phenyl]benzotriazole, 2,2'-methylenebis[6-(2H-benzotriazol-2-yl)-4-(1,1,3,3-tetramethylbutyl)phenol, 2,2'-methylenebis[4-t-butyl-6-(2H-benzotriazol-2-yl)phenol], a condensate of poly(3-11)(ethyleneglycol) with 2-[3'-t-butyl-2'-hydroxy-5'-(2-methoxycarbonylethyl)phenyl] benzotriazole, a condensate of poly(3-11)(ethyleneglycol) with methyl 3-[3-(2H-benzotriazol-2-yl)-5-t-butyl-4-hydroxyphenyl]propionate, 2-ethylhexyl 3-[3-t-butyl-5-(5-chloro-2H-benzotriazol-2-yl)-4-hydroxyphenyl]propionate, octyl 3-[3-t-butyl-5-(5-chloro-2H-benzotriazol-2-yl)-4-hydroxyphenyl]propionate, methyl 3-[3-t-butyl-5-(5-chloro-2H-benzotriazol-2-yl)-4-hydroxyphenyl]propionate, 3-[3-t-butyl-5- (5-chloro-2H-benzotriazol-2-yl)-4-hydroxyphenyl]propionic acid and any mixtures thereof.

As the light stabilizer, for example, the following can be mentioned. As the light stabilizer, the following compounds may be used alone or in combination of two or more.

### (1) Examples of hindered amine light stabilizers

Bis(2,2,6,6-tetramethyl-4-piperidyl)sebacate, bis(2,2,6,6-tetramethyl-4-piperidyl)succinate, bis(1,2,2,6,6-pentamethyl-4-piperidyl)sebacate, bis(N-octoxy-2,2,6,6-tetramethyl-4-piperidyl)sebacate, bis(N-benzyloxy-2,2,6,6-tetramethyl-4-piperidyl)sebacate, bis(N-cyclohexyloxy-2,2,6,6-tetramethyl-4-piperidyl)sebacate, bis1,2,2,6,6-pentamethyl-4-piperidyl) 2-(3,5-di-t-butyl-4-hydroxybenzyl)-2-butylmalonate, bis(1-acroyl-2,2,6,6-tetramethyl-4-piperidyl) 2,2-bis(3,5-di-t-butyl-4-hydroxybenzyl)-2-butylmalonate, bis(1,2,2,6,6-pentamethyl-4-piperidyl)decanedioate, 2,2,6,6-tetramethyl-4-piperidyl methacrylate, 4-[3-(3,5-di-t-butyl-4-hydroxyphenyl)propionyloxy]-1-[2-(3-(3,5-di-t-butyl-4-hydroxyphenyl)propionyloxy)ethyl]-2,2,6,6-tetramethylpiperidine, 2-methyl-2-(2,2,6,6-tetramethyl-4-piperidyl)amino-N-(2,2,6,6-tetramethyl-4-piperidyl)propionamide, tetrakis(2,2,6,6-tetramethyl-4-piperidyl) 1,2,3,4-butanetetracarboxylate, tetrakis(1,2,2,6,6-pentamethyl-4-piperidyl) 1,2,3,4-butanetetracarboxylate, a mixed esterified product of 1,2,3,4-butanetetracarboxylic acid with 1,2,2,6,6-pentamethyl-4-piperidinol and 1-tridecanol,
a mixed esterified product of 1,2,3,4-butanetetracarboxylic acid with 2,2,6,6-tetramethyl-4-piperidinol and 1-tridecanol, a mixed esterified product of 1,2,3,4-butanetetracarboxylic acid with 1,2,2,6,6-pentamethyl-4-piperidinol and 3,9-bis(2-hydroxy-1,1-dimethylethyl)-2,4,8,10-tetraoxaspiro [5.5]undecane, a mixed esterified product of 1,2,3,4-butanetetracarboxylic acid with 2,2,6,6-tetramethyl-4-piperidinol and 3,9-bis(2-hydroxy-1,1-dimethylethyl)-2,4, 8,10-tetraoxaspiro[5.5]undecane, a polycondensate of dimethyl succinate with 1-(2-hydroxyethyl)-4-hydroxy-2,2,6,6-tetramethylpiperidine, poly[(6-morpholino-1,3,5-triazine-2,4-diyl)((2,2,6,6-tetramethyl-4-piperidyl)imino)hexamethylene((2,2,6,6-tetramethyl-4-piperidyl)imino)], poly[(6-(1,1,3,3-tetramethylbutyl)imino-1,3,5-triazine-2,4-diyl((2,2,6,6-tetramethyl-4-piperidyl)imino)hexamethylene((2,2,6,6-tetramethyl-4-piperidyl)imino)], a polycondensate of N,N'-bis(2,2,6,6-tetramethyl-4-piperidyl)hexamethylenediamine with 1,2-dibromoethane, N,N',4,7-tetrakis[4,6-bis(N-butyl-N-(2,2,6,6-tetramethyl-4-piperidyl)amino)-1,3,5-triazine-2-yl]-4,7-diazadecane-1,10 diamine, N,N',4-tris[4,6-bis(N-butyl-N-(2,2,6,6-tetramethyl-4-piperidyl)amino)-1,3,5-triazin-2-yl]-4,7-diazadecane-1,10-diamine, N,N',4,7-tetrakis[4,6-bis(N-butyl-N-(1,2,2,6,6-pentamethyl-4-piperidyl)amino)-1,3,5-triazin-2-yl] -4,7-diazadecane-1,10-diamine, N,N',4-tris[4,6-bis(N-butyl-N-(1,2,2,6,6-pentamethyl-4-piperidyl)amino)-1,3,5-triazin-2-yl]-4,7-diazadecane-1,10-diamine and any mixtures thereof.

### (2) Examples of acrylate-based light stabilizers

Ethyl α-cyano-β,β-diphenyl acrylate, isooctyl α-cyano-β,β-diphenyl acrylate, methyl α-carbomethoxycinnamate, methyl α-cyano-β-methyl-p-methoxycinnamate, butyl α-cyano-β-methyl-p-methoxycinnamate, methyl α-carbomethoxy-p-methoxycinnamate and N-(β-carbomethoxy-β-cyanovinyl)-2-methylindoline and any mixtures thereof.

### (3) Examples of nickel-based light stabilizers

Nickel complexes of 2,2'-thiobis-[4-(1,1,3,3-tetramethylbutyl)phenol], nickeldibutyldithiocarbamate, nickel salts of monoalkyl esters, nickel complexes of ketoximes and any mixtures thereof.

### (4) Examples of oxamide-based light stabilizers

4,4'-dioctyloxyoxanilide, 2,2'-diethoxyoxanilide, 2,2'-dioctyloxy-5,5'-di-t-butylanilide, 2,2'-didodecyloxy-5,5'-di-t-butylanilide, 2-ethoxy-2'-ethyloxanilide, N,N'-bis(3-dimethylaminopropyl)oxamide, 2-ethoxy-5-t-butyl-2'-ethoxyanilide, 2-ethoxy-5,4'-di-t-butyl-2'-ethyloxanilide and any mixtures thereof.

### (5) Examples of 2-(2-hydroxyphenyl)-1,3,5-triazine light stabilizers

2,4,6-Tris(2-hydroxy-4-octyloxyphenyl)-1,3,5-triazine, 2-(2-hydroxy-4-octyloxyphenyl)-4,6-bis(2,4-dimethylphenyl)-1,3,5-triazine, 2-[2,4-dihydroxyphenyl-4,6-bis(2,4-dimethylphenyl]-1,3,5-triazine, 2,4-bis(2-hydroxy-4-propyloxyphenyl)-6-(2,4-dimethylphenyl)-1,3,5-triazine, 2-(2-hydroxy-4-octyloxyphenyl)-4,6-bis(4-methylphenyl)-1,3,5-triazine, 2-(2-hydroxy-4-dodecyloxyphenyl)-4,6-bis(2,4-dimethylphenyl)-1,3,5-triazine, 2-[2-hydroxy-4-(2-hydroxy-3-butyloxypropoxy)phenyl]-4,6-bis(2,4-dimethylphenyl)-1,3,5-triazine, 2-[2-hydroxy-4-(2-hydroxy-3-octyloxypropoxy)phenyl]-4,6-bis(2,4-dimethylphenyl)-1,3,5-triazine and any mixtures thereof.

Examples of the metal deactivator include the followings.

N,N'-diphenyloxamide, N-salicylal-N'-salicyloylhydrazine, N,N'-bis(salicyloyl)hydrazine, N,N'-bis(3,5-di-t-butyl-4-hydroxyphenylpropionyl)hydrazine, 3-salicyloylamino-1,2,4-triazole, bis(benzylidene)oxalyldihydrazide, oxanilide, isophthaloyldihydrazide, sebacoylbisphenylhydrazide, N,N'-bis(salicyloyl)oxalyldihydrazide, N,N'-bis(salicyloyl)thiopropionyldihydrazide and any mixtures thereof.

Examples of the peroxide scavenger include esters of β-thiodipropionic acid, mercaptobenzimidazole, zinc salt of 2-mercaptobenzimidazole, zinc salt of dibutyldithiocarbamic acid, dioctadecyl disulfide, pentaerythritol tetrakis(β-dodecylmercapto)propionate, and any mixtures thereof.

Examples of the polyamide stabilizer include copper or divalent manganese salt of iodide or phosphorus compound, and any mixtures thereof.

Examples of the hydroxyamine include N,N-dibenzylhydroxyamine, N,N-diethylhydroxyamine, N,N-dioctylhydroxyamine, N,N-dilaurylhydroxyamine, N,N-ditetradecylhydroxylamine, N,N-dihexadecylhydroxyamine, N,N-dioctadecylhydroxyamine, N-hexadecyl-N-octadecylhydroxyamine, N-heptadecyl-N-octadecylhydroxyamine and any mixtures thereof.

Examples of the neutralizing agent include calcium stearate, zinc stearate, magnesium stearate, hydrotalcite (basic magnesium aluminum hydroxy carbonate hydrate), melamine, amine, polyamide, polyurethane, and any mixtures thereof.

Examples of the lubricant include aliphatic hydrocarbons such as paraffin and wax, higher fatty acids having 8 to 22 carbon atoms, higher fatty acid metal (Al, Ca, Mg, Zn) salts having 8 to 22 carbon atoms, aliphatic alcohols having 8 to 22 carbon atoms, polyglycols, esters of a higher fatty acid having 4 to 22 carbon atoms with an aliphatic monohydric alcohol having 4 to 18 carbon atoms, higher aliphatic amides having 8 to 22 carbon atoms, silicone oils, rosin derivatives.

Examples of the nucleating agent include the followings. Sodium 2,2'-methylenebis(4,6-di-t-butylphenyl)phosphate, [phosphate-2,2'-methylenebis(4,6-di-t-butylphenyl)]dihydroxyaluminum, bis[phosphate-2,2'-methylenebis(4,6-di-t-butylphenyl)]hydrooxyaluminum, tris[phosphate-2,2'-methylenebis(4,6-di-t-butylphenyl)]aluminum, sodium bis(4-t-butylphenyl)phosphate, metal salts of benzoic acid such as sodium benzoate, aluminum p-t-butylbenzoate, 1,3:2,4-bis(O-benzylidene)sorbitol, 1,3:2,4-bis(O-methylbenzylidene)sorbitol, 1,3:2,4-bis(O-ethylbenzylidene)sorbitol, 1,3-O-3,4-dimethylbenzylidene-2,4-O-benzylidene sorbitol, 1,3-O-benzylidene-2,4-O-3,4- dimethylbenzylidene sorbitol, 1,3:2,4-bis(O-3,4-dimethylbenzylidene)sorbitol, 1,3-O-p-chlorobenzylidene-2,4-O-3,4-dimethylbenzylidenesorbitol, 1,3-O-3,4-dimethylbenzylidene-2,4-O-p-chlorobenzylidenesorbitol, 1,3:2,4-bis(O-p-chlorobenzylidene)sorbitol and any mixtures thereof.

Examples of the filler include calcium carbonate, silicate, glass fiber, asbestos, talc, kaolin, mica, barium sulfate, carbon black, carbon fiber, zeolite, and any mixtures thereof.

Among these additives, preferred are phenolic antioxidants, phosphorus antioxidants, ultraviolet absorbers, hindered amine light stabilizers, peroxide scavengers and neutralizing agents.

Examples of particularly preferred phenolic antioxidant include the following compounds.

2,6-Di-t-butyl-4-methylphenol, 2,4,6-tri-t-butylphenol, 2,4-dioctylthiomethyl-6-methylphenol, 2,2'-thiobis(6-t-butylphenol), 4,4'-thiobis(3-methyl-6-t-butylphenol), 2,2'-methylenebis(4-methyl- 6-t-butylphenol), 2,2'-methylenebis(4-ethyl-6-t-butylphenol), 2,2'-methylenebis[4-methyl-6-(α-methylcyclohexyl)phenol]], 2,2'-methylenebis(4-methyl-6-cyclohexylphenol), 2,2'-methylenebis(4,6-di-t-butylphenol), 2,2'-ethylidenebis(4,6-di-t-butylphenol), 4,4'-methylenebis(6-t-butyl-2-methylphenol), 4,4'-methylenebis(2,6- di-t-butylphenol), 4,4'-butylidenebis(3-methyl-6-t-butylphenol), 1,1-bis(4-hydroxyphenyl)cyclohexane, 1,1-bis(5-t-butyl-4-hydroxy-2-methylphenyl)butane, 1,1,3- tris(5-t-4-hydroxy-2-methylphenyl)butane, ethylene glycol bis[3,3-bis-3'-t-butyl-4'-hydroxyphenyl)butyrate], 2-t-butyl-6-(3'-t-butyl-5'-methyl-2'-hydroxybenzyl)-4-methylphenylacrylate, 2,4-di-t-pentyl-6-[1-2-hydroxy-3,5-di-t-pentylphenyl)ethyl]phenylacrylate,

2,4,6-tris(3,5-di-t-butyl-4-phenoxy)-1,3,5-triazine, tris(4-t-butyl-3-hydroxy-2,6-dimethylbenzyl)isocyanurate, bis(3,5-di-t-butyl-4-hydroxybenzyl)isocyanurate, tris[2-(3',5'-di-t-butyl-4'-hydroxycinnamoyloxy)ethyl]isocyanurate, diethyl-3,5-di-t-butyl-4-hydroxybenzylphosphonate, di-n-octadecyl-3,5-di-t-butyl-4-hydroxybenzylphosphonate, calcium salt of 3,5-di-t-butyl-4-hydroxybenzylphosphonic acid monoester, n-octadecyl 3-(3,5-di-t-butyl-4-hydroxyphenyl)propionate, neopentane tetrayltetrakis(3,5-di-t-butyl-4-hydroxycinnamate), thiodiethylenebis(3,5-di-t-butyl-4-hydroxycinnamate), 1,3,5-trimethyl-2,4,6-tris(3,5-di-t-butyl-4-hydroxybenzyl)benzene, 3,6-dioxaoctamethylenebis(3,5-di-t-butyl-4-hydroxycinnamate), hexamethylenebis(3,5-di-t-butyl-4-hydroxycinnamate), triethyleneglycolbis(5-t-butyl-4-hydroxy-3-methylcinnamate), 3,9-bis[2-(3-(3-t-butyl-4-hydroxy-5-methylphenyl)propionyloxy)-1,1-dimethylethyl]-2,4,4,10-tetraoxaspiro[5.5]undecane, N,N'-bis[3-(3',5'-di-t-butyl-4'-hydroxyphenyl)propionyl]hydrazine, N,N'-bis[3-(3',5'-di-t-butyl-4'-hydroxyphenyl)propionyl]hexamethylenediamine and the like.

Examples of particularly preferable phosphorus antioxidants include the following compounds.

Tris(nonylphenyl)phosphite, tris(2,4-di-t-butylphenyl)phosphite, distearylpentaerythritoldiphosphite, bis(2,4-di-t-butylphenyl)pentaerythritoldiphosphite, bis(2,4-di-t-butyl-6-methylphenyl)pentaerythritoldiphosphite, bis(2,6-di-t-butyl-4-methylphenyl)pentaerythritoldiphosphite, tetrakis(2,4-di-t-butylphenyl)-4,4'-diphenylenediphosphonite, 2,2'-methylenebis(4,6-di-t-butylphenyl) 2-ethylhexylphosphite, 2,2'-ethylidenebis(4,6-di-t-butylphenyl)fluorophosphite, bis(2,4-dit-butyl-6-methylphenyl)ethylphosphite, 2-(2,4,6-tri-t-butylphenyl)-5-ethyl-5-butyl-1,3,2-oxaphosphorinane, 2,2',2"-nitrilo[triethyl-tris(3,3',5,5'-tetra-t-butyl-1,1'-biphenyl-2,2'-diyl)phosphite, 6-[3-(3-t-butyl-4-hydroxy-5-methylphenyl)propoxy]-2,4,8,10-tetra-t-butyldibenz [d,f] [1,3,2]dioxaphosphepin and the like.

Examples of particularly preferred ultraviolet absorbers include the following compounds.

Phenyl salicylate, 4-t-butylphenylsalicylate, 2,4-di-t-butylphenyl 3',5'-di-t-butyl-4'-hydroxybenzoate, 4-t-octylphenylsalicylate, 2,4-dihydroxybenzophenone, 2-hydroxy-4-methoxybenzophenone, 2-hydroxy-4-octoxybenzophenone, 2,2'-dihydroxy-4-methoxybenzophenone, bis(5-benzoyl-4-hydroxy-2-methoxyphenyl)methane, 2,2',4,4'-tetrahydroxybenzophenone, 2-(2-hydroxy-5-methylphenyl)benzotriazole, 2-(3',5'-di-t-butyl-2'-hydroxyphenyl)benzotriazole, 2-(5'-t-butyl-2'-hydroxyphenyl)benzotriazole, 2-(2'-hydroxy-5'-t-octylphenyl)benzotriazole, 2-(3-t-butyl-2-hydroxy-5-methylphenyl)-5-chlorobenzotriazole, 2-(3'-S-butyl-2'-hydroxy-5'-t-butylphenyl)benzotriazole, 2-(2'-hydroxy-4'-octyloxyphenyl)benzotriazole, 2-(3',5'-di-t-amyl-2'-hydroxyphenyl)benzotriazole, 2-[2'-hydroxy-3',5'-bis(α,α-dimethylbenzyl)phenyl]-2H-benzotriazole and the like.

Examples of particularly preferable light stabilizers include the following compounds.

Bis(2,2,6,6-tetramethyl-4-piperidyl)sebacate, bis(1,2,2,6,6-pentamethyl-4-piperidyl)sebacate, bis(N-octoxy-2,2,6,6-tetramethyl-4-piperidyl)sebacate, bis(N-benzyloxy-2,2,6,6-tetramethyl-4-piperidyl)sebacate, bis(N-cyclohexyloxy-2,2,6,6-tetramethyl-4-piperidyl) sebacate, bis(1,2,2,6,6-pentamethyl-4-piperidyl) 2-(3,5-di-t-butyl-4-hydroxybenzyl)-2-butylmalonate, bis(1-acroyl-2,2,6,6-tetramethyl-4-piperidyl) 2,2-bis(3,5-di-t-butyl-4-hydroxybenzyl)-2-butylmalonate, bis(2,2,6,6-tetramethyl-4-piperidyl)succinate, 2,2,6,6-tetramethyl-4-piperidylmethacrylate, 4-[3-(3,5-di-t-butyl-4-hydroxyphenyl)propionyloxy]-1-[2-(3-(3,5-di-t-butyl-4-hydroxyphenyl)propionyloxy)ethyl]-2,2,6,6-tetramethylpiperidine, 2-methyl-2-(2,2,6,6-tetramethyl-4-piperidyl)amino-N-(2,2,6,6-tetramethyl-4-piperidyl)propionamide, tetrakis(2,2,6,6-tetramethyl-4-piperidyl) 1,2,3,4-butanetetracarboxylate,
tetrakis(1,2,6,6-pentamethyl-4-piperidyl) 1,2,3,4-butanetetracarboxylate, a mixed esterified product of 1,2,3,4-butanetetracarboxylic acid with 1,2,2,6,6-pentamethyl-4-piperidinol and 1-tridecanol, a mixed esterified product of 1,2,3,4-butanetetracarboxylic acid with 2,2,6,6-tetramethyl-4-piperidinol and 1-tridecanol, a mixed esterified product of 1,2,3,4-butanetetracarboxylic acid with 1,2,2,6,6-pentamethyl-4-piperidinol and 3,9-bis(2-hydroxy-1,1-dimethylethyl)-2,4,8,10-tetraoxaspiro [5.5]undecane, a mixed esterified product of 1,2,3,4-butanetetracarboxylic acid with 2,2,6,6-tetramethyl-4-piperidinol and 3,9-bis(2-hydroxy-1,1-dimethylethyl)-2,4,8,10-tetraoxaspiro[5.5]undecane, a polycondensate of dimethyl succinate with 1-(2-hydroxyethyl)-4-hydroxy-2,2,6,6-tetramethylpiperidine, poly[(6-morpholino-1,3,5-triazine-2,4-diyl)((2,2,6,6-tetramethyl-4-piperidyl)imino)hexamethylene((2,2,6,6-tetramethyl-4-piperidyl)imino)], poly[(6-(1,1,3,3-tetramethylbutyl)-1,3,5-triazine-2,4-diyl)((2,2,6,6-tetramethyl-4-piperidyl)imino)hexamethylene((2,2,6,6-tetramethyl-4-piperidyl)imino)] and the like.

The phenolic compound of the present invention that is represented by Formula (1) and/or other additives to be added as required can be added to an organic material using any known method and apparatus for obtaining a homogeneous mixture. For example, when the organic material is a solid polymer, the phenolic compound of the present invention and/or the other additives to be added as required may be directly dry-blended with the solid polymer, or the phenolic compound and/or the other additives to be added as required may be added to the solid polymer in the form of a masterbatch. When the organic material is a liquid polymer, in addition to the above-described addition method, the phenolic compound of the present invention and/or the other additives to be added as required may be, in the form of a solution or a dispersion, blended into the polymer solution during or immediately after polymerization. Meanwhile, when the organic material is a liquid (e.g., oil) other than a solid polymer, in addition to the above-described addition method, the phenolic compound of the present invention and/or the other additives to be added as required may be directly added to and dissolved in the organic material, or the phenolic compound of the present invention and/or the other additives to be added as required may be added in a state of being dissolved or suspended in a liquid medium.

The phenolic compound of the present invention that is represented by Formula (1) exhibits excellent performance as a stabilizer of various organic materials including thermoplastic resins such as polyolefins. An organic material to which the phenolic compound of the present invention is added is stable against thermal deterioration, oxidative deterioration, and the like during its production, processing, and use, and yields a high-quality product.

### EXAMPLES

The present invention will now be described in more detail by way of Examples; however, the present invention is not limited by the below-described Examples.

### Measurement of ¹H-NMR

Apparatus: AV-300M, manufactured by Broker AXS GmbH
Measurement solvent: CDCl₃

### Measurement of MFR

The MFR was measured using a melt indexer (model: L246-3537, manufactured by Technol Seven Co., Ltd.) at a temperature of 230°C with a load of 21.18 N.

Decomposition of a polypropylene resin proceeds during processing with heat, and this leads to an increase in the MFR of the polypropylene resin. Therefore, the less likely the MFR of a polypropylene resin composition to which a stabilizer is added is to be increased by processing with heat, the superior is the processing stability of the resin composition.

### Measurement of Yellow Index (YI)

Pellets obtained in each of Examples and Comparative Examples were pressed using a press machine ("PEW-5040", manufactured by Kansai Roll Co., Ltd.) at a temperature of 230°C to obtain a 1 mm-thick sheet. The yellow index (YI) of the thus obtained sheet was measured using a colorimeter ("CM-3500d", manufactured by Minolta Co., Ltd.,). A smaller YI value is regarded to indicate a further inhibition of discoloration.

### Example 1: Synthesis of Phenolic Compound Represented by Formula (1) (R¹ = methyl)

SUMILIZER (registered trademark) GA-80 (chemical formula: C₄₃H₆₄O₁₀, exact mass: 740.45, molecular weight: 740.98) in an amount of 10 g (13.5 mmol), 100 mL of acetone, 1.87 g (13.5 mmol, molecular weight: 138.20) of potassium carbonate, and 1.92 g (13.5 mmol, molecular weight: 141.94) of methyl iodide were added together and stirred overnight at room temperature. Since the raw material-to-product ratio was found to be approximately 8:2 by LC, potassium carbonate and methyl iodide were added in an amount of 1 equivalent each, and the resultant was stirred for 6 hours at room temperature. Potassium carbonate and methyl iodide were further added in an amount of 1 equivalent each, and the resultant was stirred overnight and then filtrated, after which the resulting residue was concentrated to obtain a crude product. This residue was purified by silica gel chromatography (developing solvent: hexane/ethyl acetate), whereby 4.5 g of a compound (1) represented by the following Formula (1-MeO) and 2.9 g of a dimethoxy form (DMeO) represented by the following Formula (DMeO) were obtained. The compound (1) represented by Formula (1-MeO) is the phenolic compound of the present invention. The thus obtained dimethoxy form was used in a comparative resin composition in the below-described Comparative Example 3. It is noted here that the compound (1) represented by Formula (1-MeO) had a chemical formula of C₄₄H₆₆O₁₀, an exact mass of 754.47, and a molecular weight of 755.00. The dimethoxy form represented by Formula (DMeO) had a chemical formula of C₄₅H₆₈O₁₀, an exact mass of 768.48, and a molecular weight of 769.03.

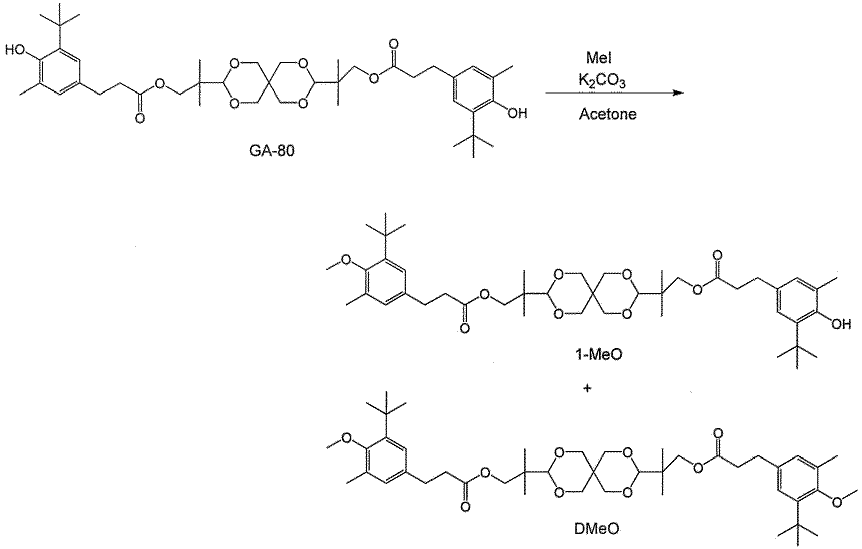

### Purity (LC)

Model: LC-20A
Column: L-column2 ODS (5 µm, 4.6 mmcp, 150 mm)
Injection amount: 5 µL (each methanol solution)
Mobile phase:
   Liquid A: water/acetic acid = 1,000/1 (v/v)
   Liquid B: methanol/THF/acetic acid = 930/70/1 (v/v/v)
Gradient: Time (min.) 0, 7, 37, 47
B conc. (%): 70, 70, 100, 100
Flow rate: 1.0 mL/min
Column temperature: 40°C
Measurement wavelength: UV, 280 nm
Purity: 99.3% (1-MeO), 97.4% (DMeO)
¹H-NMR (300 MHz, CDCl₃)
δ0.91 (d,J=1.8 Hz, 12H,CH₃), δ1.37 (s,9H,C(CH₃)₃), δ1.40 (s,9H,C(CH₃)₃), δ2.22 (s,3H,ArCH₃), δ2.29 (s,3H,ArCH₃), δ2.57-2.64 (m,4H,CH₂), δ2.82-2.89 (m,4H,CH₂), δ3.22-3.27 (dd,J = 11.7 Hz, 1.5 Hz, 2H,CH₂), δ3.40-3.55 (m,4H,CH₂), δ3.74 (s,3H,OCH₃), δ3.92 (d,J = 2.1 Hz, 4H,COOCH₂), δ4.18 (d,J = 2.4 Hz, 2H,CH), δ4.47 (m,2H,CH₂), δ4.67 (s,1H,ArOH), δ6.83 (d,J = 1.8 Hz, 1H,Ar), δ6.88 (d,J = 2.1 Hz, 1H,Ar), δ6.96 (m,2H,Ar)

### Example 2: Preparation of Pellets of Resin Composition Containing Phenolic Compound Represented by Formula (1)

A polypropylene resin (manufactured by Sumitomo Chemical Co., Ltd., MFR: 25 g/10 min) in an amount of 100 parts by mass was dry-blended with 0.05 parts by mass of calcium stearate and 0.1 parts by mass the compound (1) obtained in Example 1. The thus obtained mixture was kneaded using a twin-screw extruder having a cylinder diameter of 32 mm (model: BTN32-S2-36-L, manufactured by PLABOR Research Laboratory of Plastics Technology Co., Ltd.) in an air atmosphere at a temperature of 230°C and a screw rotation speed of 100 rpm to obtain pellets (1) of the resin composition of the present invention.

An operation of kneading the thus obtained pellets (1) using a single-screw extruder having a cylinder diameter of 30 mm (model: VS30-28, manufactured by Tanabe Plastics Machinery Co., Lid.) in an air atmosphere at a temperature of 280°C and a screw rotation speed of 100 rpm and thereby obtaining pellets was repeated 5 times to obtain pellets (2) subjected to five kneading operations. The MFR and the YI of the thus obtained pellets (2) were measured in accordance with the above-described respective measurement methods. The thus obtained results are shown in Table 1.

### Comparative Example 1

Pellets subjected to five kneading operations were prepared in the same manner as in Example 2, except that the compound (1) was not added. The MFR and the YI of the thus obtained pellets were measured in accordance with the above-described respective measurement methods. The thus obtained results are shown in Table 1.

### Comparative Example 2

Pellets subjected to five kneading operations were prepared in the same manner as in Example 2, except that 0.1 parts by mass of GA-80 used as a raw material in Example 1 was added in place of the compound (1). The MFR and the YI of the thus obtained pellets were measured in accordance with the above-described respective measurement methods. The thus obtained results are shown in Table 1.

### Comparative Example 3

Pellets subjected to five kneading operations were prepared in the same manner as in Example 2, except that 0.1 parts by mass of the dimethoxy form obtained by the production method described in Example 1 was added in place of the compound (1). The MFR and the YI of the thus obtained pellets were measured in accordance with the above-described respective measurement methods. The thus obtained results are shown in Table 1.

**[Table 1]**

| | MFR [g/10 min] | YI |
|---|---|---|
| Example 2 (1-MeO) | 135.9 | 0.41 |
| Comparative Example 1 (no addition) | 867.3 | 0.47 |
| Comparative Example 2 (GA-80) | 94.7 | 0.58 |
| Comparative Example 3 (dimethoxy form) | 583.3 | 0.29 |

With regard to the resin composition of Example 2, the pellets (2) obtained after repeating the kneading operation five times had a low MFR, and it was thus confirmed that decomposition of the resin, which is believed to be caused by the action of heat, oxygen, and the like during processing, was inhibited. In addition, the YI was low, and it was thus confirmed that coloring of the resin was inhibited as well. On the other hand, in Comparative Examples 1 to 3 where the phenolic compound of the present invention was not added, both of the inhibition of decomposition of the resin and the inhibition of coloring of the resin were not sufficiently improved at the same time after performing the kneading operation five times.

## Claims

1. A phenolic compound, represented by the following Formula (1): 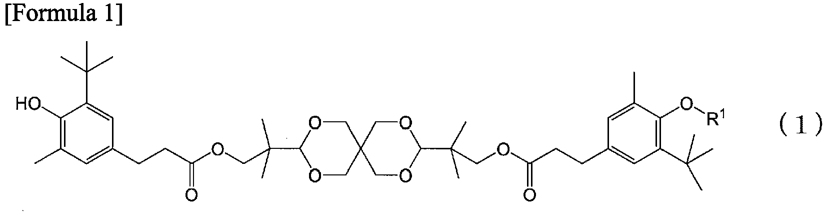 [wherein, R¹ represents an alkyl group having 1 to 2 carbon atoms].

2. The phenolic compound according to claim 1, wherein R¹ is a methyl group.

3. An organic material stabilizer, comprising the phenolic compound according to claim 1.

4. A resin composition, comprising:
the phenolic compound according to claim 1 or 2, or the organic material stabilizer according to claim 3; and
at least one organic material.

5. The resin composition according to claim 4, wherein the organic material is a thermoplastic resin.

6. The resin composition according to claim 5, wherein the thermoplastic resin is a polyolefin or an engineering plastic.

7. A method for stabilizing an organic material, comprising:
adding the phenolic compound according to claim 1 or 2, or the organic material stabilizer according to claim 3, to the organic material.

8. The method according to claim 7, wherein the organic material is a thermoplastic resin.

9. The method according to claim 8, wherein the thermoplastic resin is a polyolefin or an engineering plastic.
